Europäisches Patentamt

European Patent Office

Office européen des brevets

(11) Publication number: 0 256 629
A2

# (12) EUROPEAN PATENT APPLICATION

(21) Application number: 87305060.3

(51) Int. Cl.⁴: **A61K 31/195**

(22) Date of filing: 09.06.87

| | |
|---|---|
| The title of the invention has been amended (Guidelines for Examination in the EPO, A-III, 7.3). | (71) Applicant: **AMERICAN HOME PRODUCTS CORPORATION**<br>**685, Third Avenue**<br>**New York, New York 10017(US)** |
| (30) Priority: **12.06.86 US 873570** | (72) Inventor: **Wood, David Dudley**<br>**12 Meadow Lane**<br>**Pennington New Jersey(US)**<br>Inventor: **Mullane, John Francis**<br>**203 Cliff Avenue**<br>**Pelham New York(US)** |
| (43) Date of publication of application:<br>**24.02.88 Bulletin 88/08** | |
| (84) Designated Contracting States:<br>**AT BE CH DE ES FR GB GR IT LI LU NL SE** | (74) Representative: **Brown, Keith John Symons et al**<br>**c/o John Wyeth & Brother Limited Patent and Trademark Department Huntercombe Lane South**<br>**Taplow Maidenhead Berkshire SL6 0PH.(GB)** |

(54) **Tolrestat or a salt thereof as an immuno-stimulating agent.**

(57) A method is disclosed for stimulating the immune response in a human subject to an increased risk of infection and requiring restoration of the immune response by administering an effective amount of tolrestat or a salt thereof. Tolrestat or its salt can be used in the preparation of a medicament for stimulating the immune response.

EP 0 256 629 A2

## IMMUNO-STIMULATING AGENT

This invention relates to a novel therapeutic use of N-[[5-(trifluoromethyl)-6-methoxy-1-naphthenyl]-thioxomethyl]-N-methylglycine or a therapeutically acceptable salt thereof. More specifically this invention relates to a method for stimulating the immune response in humans subject to an increased risk of infection and requiring restoration of the immune response.

The active agent of this invention, N-[[5-(trifluoromethyl)-6-methoxy-1-naphthenyl]-thioxomethyl]-N-methylglycine or a therapeutically acceptable salt thereof, is disclosed in U.S.Patent No: 4,568,693 and European Patent No: 59596. This active agent, hereinafter designated by its generic name tolrestat, previously has been reported to be useful in preventing or relieving diabetic complications such as cataracts, neuropathy, nephropathy and retinopathy (See U.S. Patent No: 4,568,693). We have now found unexpectedly that tolrestat, either in its free acid form or in its therapeutically acceptable salt form, is useful for stimulating the immune response in humans, requiring restoration of the immune response and particularly humans suffering from diabetes mellitus.

This finding, coupled with the fact that tolrestat is a relatively safe drug, renders the method of this invention particularly useful and advantageous.

According to this invention a method is provided for stimulating the immune response in a human, e.g. one subject to an increased risk of infection, in need of restoration of said immune response which comprises administering to the human an effective amount of tolrestat, or a therapeutically acceptable salt thereof.

In another aspect the present invention provides the use of tolrestat or a therapeutically acceptable salt thereof for the preparation of a medicament for stimulating the immune response in a human requiring restoration of the immune response.

According to the present invention tolrestat, either in its free acid form or in the therapeutically acceptable salt form, is employed as the active agent. Example of suitable salt forms are described in U.S. Patent No: 4,568,693 and include the sodium, potassium, magnesium, triethylamine and benzylamine salt forms. A preferred salt form is the sodium salt, i.e. tolrestat sodium.

Tolrestat or a therapeutically acceptable addition salt thereof may be administered to humans subject to an increased risk of infection either orally or parenterally. For many reasons oral administration is prefered.

While tolrestat or a therapeutically acceptable salt thereof can be administered alone, e.g. as a sole component of a filled capsule, it is preferred to formulate the compound in various dosage forms for oral or parenteral administration, e.g. tablets, or sterile solutions. Such formulations are described in U.S.Patent No: 4,568,693 and European Patent 59596 herein incorporated by reference in their entirety.

When utilising tolrestat or one of its above-noted salts as an immuno-moderator, the total dose of active agent can range, for example, from 0.1 to 20 mg per kilogram of body weight per day with a preferred dosage range of from 100 to 400 milligrams per patient per day. Generally, a parenteral dose or an oral dose is administered in one to four applications per day. Such doses are considered to be an effective amount when, following their administration, the weight of the patient is stabilized or when the subjective symptoms complained of by said patients are reported to have been ameliorated or to have disappeared.

It is well known that humans suffering from diabetes mellitus have an increased susceptibility to and frequency of infections, such as fungal, bacterial and viral infections, due to an immunosuppressed state. The cause of this immunosuppression is as yet unknown. Although not intending to be bound by any casual theory, the immunosuppression may be related to cell damage in the cells of the immune system, including the white blood cells which fight infection, caused by the accumulation of polyols in the cells. Tolrestat is known to inhibit such accumulation of polyols in the lens, peripheral nervous cord and kidney of diabetic animals. Note the above cited U.S.Patent 4,568,693 at column 1, lines 43-50. It is believed that tolrestat also inhibits accumulation of polyols in the cells, including the white blood cells, of the immune system and consequently reverses the immuno-suppression caused by the polyols accumulation resulting in stimulation of the immune response. In any event, diabetic humans being administered tolrestat are significantly more resistant to infections than diabetic humans not being administered tolrestat.

## Claims

1. The use of tolrestat or a therapeutically acceptable salt thereof for the preparation of a medicament for stimulating the immune response in a human requiring restoration of the immune response.

2. The use of tolrestat or a therapeutically acceptable salt thereof as claimed in claim 1 for the preparation of a medicament for stimulating the immune response in humans by the administration of tolrestat or the therapeutically acceptable salt thereof at a dose of 0.1 to 20 mg per kilogram of body weight.

3. The use of tolrestat or a therapeutically acceptable salt thereof as claimed in claim 1 for the preparation of a medicament for stimulating the immune response in humans by the administration of tolrestat or the therapeutically acceptable salt thereof at a dose of 100 to 400 milligrams per day.

4. The use as claimed in any one of claims 1 to 3 in which the therapeutically acceptable salt is the sodium salt.

5. The use of tolrestat or a therapeutically acceptable salt thereof as claimed in any one of claims 1 to 4 for the preparation of a medicament for stimulating the immune response in a human suffering from diabetes mellitus.